Europäisches Patentamt

European Patent Office

Office européen des brevets

(11) Veröffentlichungsnummer: **0 546 235 A1**

## (12) EUROPÄISCHE PATENTANMELDUNG

(21) Anmeldenummer: **91890301.4**

(51) Int. Cl.5: **A61K 7/06**

(22) Anmeldetag: **09.12.91**

(43) Veröffentlichungstag der Anmeldung:
**16.06.93 Patentblatt 93/24**

(84) Benannte Vertragsstaaten:
**AT BE CH DE DK ES FR GB GR IT LI LU NL SE**

(71) Anmelder: **Lassoued, Rachida**
**7 Rue Strabon**
**Carthage Byrsa(TN)**

(72) Erfinder: **Lassoued, Rachida**
**7 Rue Strabon**
**Carthage Byrsa(TN)**

(74) Vertreter: **Köhler-Pavlik, Johann, Dipl.-Ing.**
**Margaretenplatz 5**
**A-1050 Wien (AT)**

(54) **Haarwuchsmittel.**

(57) Haarwuchsmittel aus natürlichen Bestandteilen zum Auftragen auf die Kopfhaut aus einer Mischung von Rhizinus-, Mandel-, Oliven- und vorteilhafterweise Kokosöl. Vorzugsweise werden noch Glycerin und bzw. oder Paraffinöl zugesetzt.

EP 0 546 235 A1

Die Erfindung betrifft ein Haarwuchsmittel zum Aufbringen auf die Kopfhaut aus natürlichen Bestandteilen.

Seit langer Zeit besteht immer wieder das Bedürfnis nach Mitteln und Substanzen, welche Haarausfall stoppen oder sogar den Haarwuchs wieder anregen. Die Auswirkungen schütteren Haares bzw. der Bildung kahler Stellen sind bei Personen beiderlei Geschlechts von eminenter psychologischer Bedeutung für das Selbstbewußtsein und das Lebensgefühl der Betroffenen.

Über die Gründe des Haarausfalles sind etliche Theorien aufgestellt worden, beispielsweise jene, die übermäßige Haarfettausscheidung mit daraus resultierender Störung der Hautatmung als Hauptgrund nennt. Eine andere Theorie meint, daß durch das Größerwerden des knöchernen Schädels die Kopfhaut übermäßig gespannt und dadurch die Haut mit den Haarpapillen unter Druck gesetzt wird, wodurch die versorgenden Blutgefäße abgedrückt werden.

Zur Vorbeugung von Haarausfall kommen verschiedene Maßnahmen, beispielsweise Hormon- und Vitamingaben, oder Einreibungen mit Haarölen, -cremes oder -wässern in Betracht. Auch operative Eingriffe, d.h. Verpflanzung von gesunden Haaren an kahle Stellen sind heute weit verbreitet.

Die operative Methode ist aber, abgesehen vom hohen Preis, mit den Risiken jedes chirurgischen Eingriffes behaftet und vermag oftmals die tatsächlichen Ursachen des Haarausfalles nicht zu beseitigen.

So ist beispielsweise in der DE-OS 2 145 204 ein Haartonikum beschrieben, bei welchem als Grundlage sulfoniertes Oliven- oder Rhizinusöl dient. Dieses sulfonierte Öl wird als Detergensbase verwendet und macht etwa 60-70 Vol% des Tonikums aus. Zusätzlich werden aber noch Haarnahrungsmittel wie Schwefel, ein Haarwuchsstimulans, beispielsweise Acetylsalicylsäure, Korrekturmittel, Proteine usw. verwendet. Für dieses Tonikum sind also relativ viele Komponenten notwendig, welche noch dazu in unterschiedlichen Anteilen beigemengt werden müssen. Weiters ist je nach der Ursache des Haarausfalls vorgesehen, die Zusammensetzung etwas abzuändern. Damit ist also neben einer aufwendigeren, weil genaueren Herstellung auch die Verwendung von unterschiedlichen Stoffgruppen vorgesehen.

Dies trifft auch für die haarwuchsfördernde Zusammensetzung gemäß der AU-PS 440 609 zu. Neben pflanzlichen Ölen als bindende und verdickende Basis sind als wirksame Substanzen ein wässriger Auszug aus Krokusknollen, Salicylsäure und Schwefelniederschlag vorgesehen. Auch hier werden die einzelnen Komponenten in unterschiedlichen Mengen zugegeben und bedürfen darüberhinaus spezieller Herstellungsverfahren. Insbesondere trifft dies für die Herstellung des wässrigen Auszuges aus den Krokusknollen zu, welcher den Wirkstoff der Zusammensetzung bildet.

In der DE-OS 33 01 158 ist schließlich ein Haarwuchsmittel beschrieben, welches aus naturbelassenen pflanzlichen Ölen sowie Kamillenblütenextrakt besteht. Während zwei dieser Öle in gleicher Menge verwendet werden, wird das dritte pflanzliche Öl in geringerer Menge beidosiert und die so entstehende Mischung wird anschließend mit Kamillenblüten vermischt und zum Extrahieren von deren Wirkstoffen einmal bis zum Sieden erhitzt und anschließend etwas da Ziehen überlassen. Danach muß abfiltriert und bis zum Erkalten gewartet werden. Hier ist wiederum eine unterschiedliche Dosierung der einzelnen Komponenten des Haarwuchsmittels notwendig und darüberhinaus ist auch das Herstellungsverfahren durch den Schritt der Extrahierung der Kamillenblüten relativ aufwendig.

Schließlich ist auch anzumerken, daß in keinem der bislang genannten Haarwuchsmittel bzw. Haartonika die günstige Wirkung von Mandelöl erwähnt ist.

Für viele der derzeit angebotenen Präparate, ob sie nun chemischsynthetisch oder auf natürlicher Basis, beispielsweise aus Kräuterauszügen od. dgl. hergestellt sind gilt, daß sie die tatsächlichen Ursachen des Haarausfalles nicht beseitigen können. Bei etlichen dieser Produkte setzt der Haarausfall nach Stoppen der Anwendung wieder ein, falls überhaupt Besserung erzielt werden konnte.

Die Aufgabe der Erfindung war daher, die Entwicklung eines Haarwuchsmittels auf natürlicher, vorzugsweise pflanzlicher Grundlage, welches Haarausfall verläßlich stoppt, das Wachsen kräftiger gesunder Haare anregt und die Kopfhaut pflegt und schützt. Dabei soll aber eine möglichst einfach Herstellung gewährleistet sein, ohne daß komplizierte Dosierungen der einzelnen Komponenten bzw. langwierige Vorbereitungs- und Herstellungsprozesse nötig sind.

Dazu ist das Haarwuchsmittel gemäß der Erfindung dadurch gekennzeichnet, daß es eine Mischung aus Rhizinus-, Mandel- und Olivenöl ist, wobei diese pflanzlichen Öle bzw. Fette vorzugsweise zu gleichen Teilen in der Mischung vorliegen.

Das erfindungsgemäße Präparat stoppt bei Anwendung über etwa zwei bis zwölf Wochen, abhängig von der jeweiligen Hautbeschaffenheit und der Ursache, den Haarausfall und sorgt für die Anregung des Haarwuchses, sodaß kahle Stellen verschwinden und schütteres Haar dichter wird. Dabei sind die aufgrund der Anwendung des erfindungsgemäßen Präparates nachwachsenden Haare von kräftiger Beschaffenheit. Schließlich kommt es auch zu einer günstigen Auswirkung auf die Kopfhaut mit Beseitigung von Schuppen und Verhinderungen von deren Neubildung.

In der folgenden Beschreibung sollen weitere Merkmale und Eigenschaften der Erfindung ausführlich dargestellt werden.

Der wesentliche Bestandteil des erfindungsgemäßen Präparates ist eine Mischung von mindestens drei pflanzlichen Ölen bzw. Fetten mit stark unterschiedlichen Verseifungs- und Jodzahlen, nämlich Rhizinus-, Mandel- und Olivenöl. Um in weiterer Folge die Doppelbezeichnung "Fette und Öle" zu vermeiden, soll unabhängig vom Aggregatzustand des Stoffes bei einer beliebigen Temperatur lediglich der Ausdruck "Öl" verwendet werden, da die vorzugsweise verwendeten Substanzen im wesentlichen bei Zimmertemperatur flüssig vorliegen. Wie hinlänglich bekannt ist, sind pflanzliche Öle und Fette chemisch gesehen Fettsäureglycerinester. Da Glycerin ein 3-wertiger Alkohol ist, kommen vielfach gemischte Ester vor, d.h. es sind zwei oder drei verschiedene Fettsäuren an einem Glycerinmolekül gebunden. Überdies können in den besagten Ölen noch andere Substanzen, wie beispielsweise die Phytosterine (hochmolekulare aromatische Alkohole) vorhanden sein.

Unter der zuvor angesprochenen Verseifungszahl versteht man die Menge reinen Kaliumhydroxids in mg (in gelöstem Zustand angewendet), die zur völligen Verseifung von 1g Öl verbraucht werden. Die Verseifungszahl bietet also einen Anhaltspunkt für die durchschnittliche Molekülgröße der beteiligten Fettsäuren. Je höher sie ist, desto mehr niedermolekulare, flüchtige Fettsäuren liegen vor. Der numerische Bereich der Verseifungszahlen reicht von 170 - 180,(z.B. für Rhizinus- oder Traubenkernöl) über mittlere Werte von 190 - 195 (etwa bei Oliven-, Mandel- und Sonnenblumenöl) bis zu 205 - 290 (beispielsweise 258 - 264 für Kokosöl).

Die Jodzahl wiederum gibt an, wieviel g Jod von 100g Fett unter Entfärbung chemisch gebunden werden und liefert damit einen Vergleichsmaßstab für die Anzahl an ungesättigten, d.h. mit Doppelbindungen versehenen Fettsäuren. Hier reichen die Zahlenwerte von unter 10 (Kokosöl 7,5 - 9,4) bis zu Werten von 120 - 200 (z.B. Leinöl, Mohnöl).

Die Mischung aus Rhizinus-, Mandel-und Olivenöl zeigt bereits gute Wirkung im Sinne der gestellten Aufgabe und folgende Tabelle gibt deren Eigenschaften wieder:

| Bezeichnung | Dichte | Fp. | Verseifungs-zahl | Jodzahl |
|---|---|---|---|---|
| Rhizinusöl (Oleum Ricini) | 0,95-0,97 | -10°bis-12°C | 176-190 | 82-90 |
| Mandelöl (Oleum Amygdalarum dulcium) | 0,91-0,92 | - 10°C | 190-195 | 95-100 |
| Olivenöl (Oleum Olivarum) | 0,91-0,92 | - 6°C | 185-203 | 75-94 |

Alle der drei genannten Öle sind, einzeln verwendet, für kosmetische und medizinische Anwendungen bekannt. Dies trifft auch auf ein weiteres pflanzliches Öl zu, welches nach einem weiteren Merkmal der Erfindung vorteilhafterweise zugesetzt wird. Es handelt sich dabei um Kokosöl (Oleum Cocos) mit einer Dichte von 0,88 bis 0,9, einem Schmelzpunkt von 20 bis 23 ° C, einer Verseifungszahl zwischen 255 - 260 und einer Jodzahl von 7,5 - 9,5. Darüberhinaus enthält die Kokosmilch, welche in den zuletzt harten, fettreichen Kern übergeht, Spuren von Wuchsstoffen (z.B. 0,1 ppm Diphenylharnstoff).

Wenn, wie dies erfindungsgemäß vorzugsweise vorgesehen ist, die pflanzlichen Öle in der Mischung zu gleichen Teilen vorliegen, kommen deren Eigenschaften am ausgewogensten zur Geltung und ergeben optimale Resultate für die jeweils verwendeten Ölarten. Das Mischungsverhältnis ist dabei volumetrisch bestimmt.

Ein Präparat aus den zuvor ausführlich vorgestellten vier genannten Ölen ergab im Versuch die besten Resultate, wobei bei einer Anwendungsdauer von jeweils zwei bis 24 Stunden pro Auftragung, mit anschließendem gründlichen Ausspülen, über einen längeren Zeitraum (zwei bis sechs Wochen) auch bei Personen mit völlig kahlen Stellen die Haare wieder nachwuchsen. Je länger die Anwendungsdauer pro Auftragung ausgedehnt wird, umso dichter, kräftiger und schneller wächst das Haar. Vorzugsweise sollte die Behandlung zweimal wöchentlich vor dem Schlafengehen vorgenommen werden, wobei eine entsprechende Menge des Präparates nach Schütteln auf die Kopfhaut aufgetragen und leicht massierend verteilt und mit den Fingerkuppen eingerieben wird. Das Haar wird erst am Morgen nach der Behandlung mit guter Seife gewaschen. Je nach individuellem Bedarf wird die Behandlung mindestens sechs Wochen lang durchgeführt oder erst nach Stärkung des neuen Haarwuchses unterbrochen.

Auch bei Absetzen der Behandlung bleibt die anregende und haarwuchsfördernde Wirkung meist über Zeiträume von mindestens einem halben Jahr erhalten. Vermutlich sind synergetische Effekte für diese Wirkungen verantwortlich.

Schließlich können der oben beschriebenen Mischung der pflanzlichen Öle noch weitere Stoffe zugegeben werden. Dabei handelt es sich vorzugsweise um Glycerin (Propantriol) und Paraffinöl (Vaselinöl, Paraffinum liquidum). Diese beiden Substanzen können jede für sich zugesetzt werden, vorteilhafterweise sind beide in der bevorzugten Variante des erfindungsgemäßen Haarwuchsmittels vorhanden. Glycerin hat aufgrund seiner hygroskopischen Eigenschaft die Wirkung eines feuchtigkeitserhaltenden Zusatzes, und das Paraffinöl dient zur Verdünnung der in den pflanzlichen Ölen enthaltenen Wirkstoffe.

Als bevorzugte Zusammensetzung mit der besten haarkräftigenden und wuchsfördernden Wirkung hat sich als Konsequenz obiger Ausführungen, von Versuchen in der Praxis bestätigt, folgende Mischung erwiesen: zu volumetrisch gleichen Teilen Mandel-, Rhizinus-, Oliven- und Kokosöl, sowie Glycerin und Paraffinöl. Die Mischung bleibt bei geschlossener Flasche mindeste sechs Monate stabil und gebrauchsfertig.

Ausführungsbeispiel:

Eine Mischung aus je 1/6 Volumsanteilen Rhizinus-, süßes Mandel-, Oliven-, Kokosöl, Glycerin und Paraffin wurde zweimal wöchentlich auf eine vom Haarausfall befallene und Schuppen aufweisende Kopfhaut aufgetragen und jeweils durch drei Minuten leicht einmassiert. Der Haarausfall hörte nach Anwendung der Lotion nach 15 Tagen auf. In der gleichen Zeit sind auch die Schuppen verschwunden. Die ersten Haare haben etwa 40 Tage nach Anwendung der Lotion zu wachsen begonnen.

Die Lotion hat auch das Wachstum der Haare beschleunigt.

Außerdem wurde festgestellt, daß die Lotion am Kopf nicht länger als 24 Stunden verbleiben soll.

**Patentansprüche**

1.  Haarwuchsmittel zum Aufbringen auf die Kopfhaut aus natürlichen Bestandteilen, dadurch gekennzeichnet, daß es eine Mischung aus Rhizinus-, Mandel- und Olivenöl ist, wobei diese pflanzlichen Öle bzw. Fette vorzugsweise zu gleichen Teilen in der Mischung vorliegen.

2.  Haarwuchsmittel nach Anspruch 1, dadurch gekennzeichnet, daß zusätzlich Kokosöl zugesetzt ist.

3.  Haarwuchsmittel nach einem der vorangehenen Ansprüche, dadurch gekennzeichnet, daß Glycerin und/oder Paraffinöl zugesetzt ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | DE-A-3 633 242 (ARTIRAN) <br> * das ganze Dokument * <br> --- | 1-3 | A61K7/06 |
| X | BE-A-570 171 (LABORATOIRES DES PRODUITS COSMETIQUES JEAN-MARIE) <br> * das ganze Dokument * <br> --- | 1 | |
| D,X | DE-A-2 145 204 (MAUGHAN) <br> * das ganze Dokument * <br> --- | 1-3 | |
| X | CH-A-272 709 (TEMPIA-GALIERA) <br> * das ganze Dokument * <br> --- | 1 | |
| Y | EP-A-0 332 382 (CLACHAR) <br> * das ganze Dokument * <br> , --- | 1-3 | |
| Y | FR-A-1 337 769 (PECK) <br> * Beispiel 6 * <br> --- | 1-3 | |
| Y | FR-A-2 167 407 (BRAQUART) <br> * das ganze Dokument * <br><br> ----- | 1-3 | **RECHERCHIERTE SACHGEBIETE (Int. Cl.5 )** <br><br> A61K |

Der vorliegende Recherchenbericht wurde für alle Patentansprüche erstellt

| Recherchenort | Abschlußdatum der Recherche | Prüfer |
|---|---|---|
| DEN HAAG | 16 JUNI 1992 | FISCHER J.P. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes
   Dokument

EPO FORM 1503 03.82 (P0403)